# EUROPEAN PATENT SPECIFICATION

(11) **EP 3 045 201 B1**
(45) Date of publication and mention of the grant of the patent: **29.11.2017**
(21) Application number: 16150353.7
(22) Date of filing: 06.01.2016
(51) Int. Cl.: A61M 27/00, A61F 2/88, A61F 2/94

(54) **URETERAL STENT WITH SIDEPORTS**
HARNRÖHRENSTENT MIT SEITENANSCHLÜSSEN
STENT URÉTÉRAL AVEC PORT LATÉRAL

(30) Priority: 13.01.2015 US 201562102697 P
(43) Date of publication of application: 20.07.2016
(73) Proprietor: Cook Medical Technologies LLC, Bloomington, IN 47404 (US)
(72) Inventor: BILTZ, Benjamin T., Spencer, IN 47460 (US)
(74) Representative: Garratt, Peter Douglas

(56) References cited:
- EP-A1- 0 672 394
- US-A1- 2004 087 886
- US-A1- 2007 276 466
- US-A1- 2013 060 238

## Description

### PRIORITY CLAIM

### BACKGROUND

### 1. Technical Field

The technical field of the invention is implantable medical devices, and in a particular example, a stent useful for urinary drainage.

### 2. Background Information

Minimally-invasive surgery may be used in the removal of stones and calculi from kidneys and ureters. To treat this condition, several individual steps are involved. In one procedure, these steps include placing a relatively narrow guidewire through a urethra and a bladder and then through the ureter and into the kidney. After the guidewire is placed, a catheter is run along the guidewire, dilating the body passages (the urethra and the ureter) as it moves down the guidewire. In the next sequence for this procedure, a ureteral access sheath is guided along and down the guidewire and the catheter. The access sheath also dilates the body passages as it moves from outside the body, through the urethra, and into the ureter, down to the desired location, and into or very near the kidney.

The physician may then remove calculi and stones through the access sheath, using a grasper, a retrieval basket, or other device. The access sheath protects the ureter from repeated passage of the retrieval device while the stones or calculi are removed. After the stones are removed, a ureteral stent may be placed into the ureter through the access sheath, using the catheter or a pushing tube to position the stent. The stent is used to retain patency of the ureteral lumen and to continue normal urinary drainage.

One problem that is encountered with ureteral stents occurs in cancer patients, where a growth may apply radial compression to a ureter. Such compression can make fluid flow difficult. In these cases, a typical polymeric, relatively soft pig-tail stent may not have sufficient radial strength to resist compression by a cancerous or other growth. In these cases, a stronger, sturdier ureteral stent is needed to resist radial compression and allow for continued drainage from the kidney to the bladder. In some cases, a urethral stent or catheter may also be helpful to ensure drainage from the bladder. EP0672394 discloses an urinary stent formed by a coilded metallic wire including a number of closely contacting spires, where at least a portion of which permit the flow of urine from the inner to the outer sides of the stent.

EP2155111 discloses an ureteral stent including a coiled wire defining a first lumen communicating outside the coil wire through small spaces between the coils; and a second coiled wire disposed within the first lumen and defining a second lumen having a diameter that may be significantly small than the diameter of the first lumen.

### BRIEF SUMMARY

The invention defines a stent according to the wording of claim 1: The stent includes a first coiled wire, which forms a first lumen defined by an inner surface collectively of each coil formed from the first coiled wire. The first coiled wire includes a distal end portion, a proximal end portion, and a central portion between the two end portions. The stent further includes a second coiled wire with an outer surface collectively of each coil formed by the second coiled wire. The second coiled wire forms a second lumen, is disposed within the first lumen of the first coiled wire, and is secured to the first coiled wire such that substantially the entire outer surface of the second coiled wire contacts substantially the entire inner surface of the first coiled wire. The second coiled wire includes a distal end portion, a proximal end portion, and a central portion between the two end portions. The first and second coiled wires are aligned with respect to each other so that the respective distal end portions, proximal end portions, and central portions of the first and second coiled wires are next to each other. The stent further includes apertures passing through each of the first and second coiled wires and into the second lumen. The apertures are located within the distal end, proximal end, and central portions of the first and second coiled wires.

A representative example of a stent not forming part of the invention is disclosed: The stent includes a first coiled wire, which forms a first lumen between a distal end portion and a proximal end portion. The first coiled wire has an inner diameter and an outer diameter. The outer diameter is tapered at the distal end portion, and the inner diameter is constant at the distal end portion. The stent further includes a second coiled wire; which forms a second lumen, is disposed within the first lumen, and is secured to the first coiled wire such that substantially all of an outer surface of the second coiled wire contacts substantially all of an inner surface of the first coiled wire.

A second representative example of a stent is disclosed: The stent includes a first coiled wire, which forms a first lumen. The first coiled wire includes a distal end portion, a proximal end portion, and a central portion between the two end portions. The stent further includes a second coiled wire with an outer surface collectively of each coil formed by the second coiled wire. The second coiled wire forms a second lumen, is disposed within the first lumen, and is secured to the first coiled wire. The second coiled wire includes a distal end portion, a proximal end portion, and a central portion between the two end portions. The first and second coiled wires are aligned with respect to each other so that the respective distal end portions, proximal end portions, and central portions of the first and second coiled wires are next to each other. The stent further includes apertures passing through each of the first and second coiled wires and into the second lumen. The apertures are located within the distal end, proximal end, and central portions of the first and second coiled wires.

Another exemplary stent is disclosed: The stent includes an outer sleeve, which forms a first lumen defined by an inner surface of the outer sleeve. The outer sleeve includes a distal end portion, a proximal end portion, and a central portion between the two end portions. The stent further includes an inner coiled wire with an outer surface collectively of each coil formed by the inner coiled wire. The inner coiled wire forms a second lumen, is disposed within the first lumen of the outer sleeve, and is secured to the outer sleeve such that substantially the entire outer surface of the inner coiled wire contacts substantially the entire inner surface of the outer sleeve. The inner coiled wire includes a distal end portion, a proximal end portion, and a central portion between the two end portions. The outer sleeve and the inner coiled wire are aligned with respect to each other so that the respective distal end portions, proximal end portions, and central portions of the outer sleeve and the inner coiled wire are next to each other. The stent further includes apertures passing through each of the outer sleeve and the inner coiled wire and into the second lumen. The apertures are located within the distal end, proximal end, and central portions of the first and second coiled wires.

The invention may also include least one pigtail defined by the distal end portion, the proximal end portion, or both end portions; a polymer coating infused with one of a pharmacological compound, a bioactive compound, or a biologic compound around an outer surface of the first coiled wire, with the apertures further passing through the polymer coating; and an outer diameter of the first coiled wire tapered at the distal end portion, the proximal end portion, or both end portions of the first coiled wire toward an end tip upon the distal end portion, the proximal end portion, or both end portions of the first coiled wire, and an inner diameter of the first coiled wire constant at the distal end portion, the proximal end portion, or both end portions of the first coiled wire.

Embodiments of the stents according to the present invention, are described herein. The accompanying drawings and descriptions are mean to be illustrative rather than limiting.

### BRIEF DESCRIPTION OF THE DRAWINGS

FIG. 1a shows a pigtail ureteral stent;
FIG. 1b shows a transverse cross section of an ureteral stent in a region 12 shown in FIG. 1a;
FIG. 1c shows a transverse cross section of an ureteral stent along the line 14-14' shown in FIG. 1a;
FIG. 2 shows a longitudinal cross section of a tapered tip in one embodiment of an ureteral stent;
FIG. 3 shows a longitudinal cross section of a tapered tip in another embodiment of an ureteral stent;
FIG. 4a shows a transverse cross section of a longitudinal cross section of one embodiment of an ureteral stent;
FIG. 4b shows a transverse cross section of a longitudinal cross section of another embodiment of an ureteral stent;
FIG. 4c shows a transverse cross section of a longitudinal cross section of still another embodiment of an ureteral stent; and
FIG. 5 shows a first coiled wire and a second coiled wire of an ureteral stent.

### DETAILED DESCRIPTION OF THE DRAWINGS AND THE PRESENTLY PREFERRED EMBODIMENTS

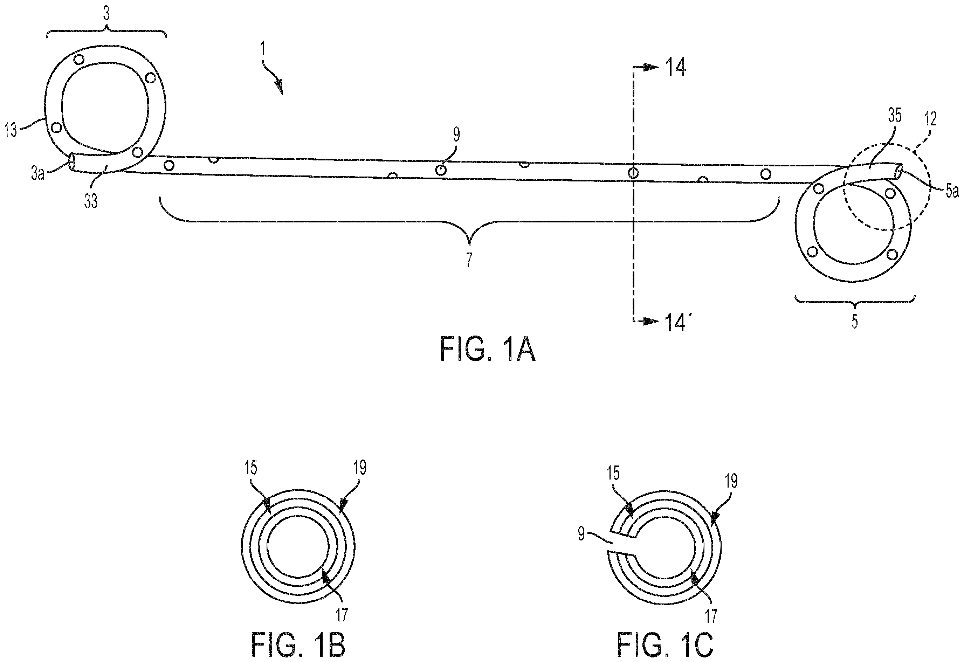

Turning now to FIGS. 1-5, an ureteral stent 1 is provided. The stent 1 will be discussed herein with respect to a design clinically configured to be used in a patient's ureter. One of ordinary skill will appreciate after a thorough review of this disclosure that the stent 1 could also be used in other clinical settings, such as for a urethra, a bile duct, a vasculature, or other clinical locations where a stent that provides patency with strong loop strength would be warranted. While the stent 1 could be readily modified by one of ordinary skill to meet the clinical requirements of other parts of the anatomy, the stent 1 for use with a patient's ureter will be discussed here for the sake of brevity.

The ureteral stent 1 includes a distal end portion 3, a proximal end portion 5, and a central portion 7 between the two end portions 3, 5. The end portions 3, 5 are open for the passage of the ureteral stent 1 over a previously inserted wireguide (not shown) to its final location within the anatomy. The ureteral stent 1 also includes a plurality of apertures 9 (or a single aperture 9) that are located within one or more of the distal end portion 3, the proximal end portion 5, and the central portion 7. The ureteral stent 1 may be constructed to be biased to define a pigtail 13 defined by the distal end portion 3, the proximal end portion 5, or both end portions 3, 5. The ureteral stent 1 also may include no pigtail 13. If the pigtail 13 is located on both end portions 3, 5, then the portion of the ureteral stent 1 between the pigtails 13 may preferably be about 4 cm to about 30 cm long. Other lengths may be used. While the pigtail 13 is depicted, one of ordinary skill in the art will understand that the ends of the ureteral stent 1 may define other shapes, such as a J, a curve, or an angle, that may be used to retain the ends of the ureteral stent 1 in the kidney and bladder, respectively.

Referring to FIGS. 1b-2, the ureteral stent 1 is made from a first coiled wire 15 and a second coiled wire 17 disposed within a first lumen 21 defined by the first coiled wire 15. The first coiled wire 15 defines the distal end portion 3, the proximal end portion 5, and the central portion 7 between the two end portions 3, 5. The first and second coiled wires 15, 17 may be manufactured from any material, and bio-compatible materials may be preferred. The first and second coiled wires 15, 17 may each be manufactured from the same material or different materials. The following materials may be preferable: 302 stainless steel, MP35N, 35N LT, CP Titanium, platinum alloys, 304V stainless steel, DFT, Ti6Al-4V ELI titanium alloy, 316L stainless steel, L-605, and nitinol.

The second coiled wire 17 is disposed within the first lumen 21 defined by the first coiled wire 15. The second coiled wire 17 defines the distal end portion 3, the proximal end portion 5, and the central portion 7 between the two end portions 3, 5, which is aligned with the respective distal and proximal end portions 3, 5 and central portion 7 of the first coiled wire 15. The second coiled wire 17 defines a second lumen 11 therewithin.

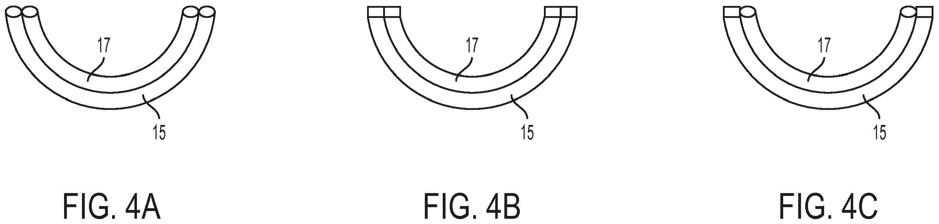

The first and second coiled wires 15, 17 are secured together such that substantially all of an outer surface 25 of the second coiled wire 17 contact substantially all of an inner surface 23 of the first coiled wire 15 so that the second coiled wire 17 may be fixedly retained within first lumen 21. In the context of the outer surface 25 of the second coiled wire 17 contacting the inner surface 23 of the first coiled wire 15, "substantially" is defined as at least 75% of the surface area of the outer surface 25 of the second coiled wire 17 contacting the inner surface 23 of the first coiled wire 15. The first and second coiled wires 15, 17 may be longitudinally fixed together. The first and second coiled wires 15, 17 may be fixed together with one or more weld beads, mechanical fasteners, adhesives, press fit, or by other methods known to fix similar materials together. In some embodiments, both of the first and second coiled wires 15, 17 may be round wires, as shown in FIG. 4a. In other embodiments, both of the first and second coiled wires 15, 17 may be flat wires, as shown in FIG. 4b. In still other embodiments, one coiled wire may be flat and the other may be round, as shown in FIG. 4c. While the first coiled wire 15 is depicted as flat and the second coiled wire 17 is depicted as round in FIG. 4c, one of ordinary skill will understand that the first coiled wire 15 could be round and the second coiled wire 17 could be flat. In some embodiments, one or both of the first and second coiled wires 15, 17 may be hollow.

The ureteral stent 1 further may be constructed to be biased to define deformable pigtails 13 defined by one or both of the distal and proximal end portions 3, 5 of the first and second coiled wires 15, 17 similar to the ureteral stent 1 shown in FIG. 1a. The pigtails 13 may help maintain the ureteral stent 1 in the selected position within the patient's anatomy in certain situations, as well as various other benefits. Because both of the distal and proximal end portions 3, 5 include respective pigtails 13, the ureteral stent 1 may resist significant longitudinal movement through the ureter due to the pigtails' 13 resistance to deformation of the pigtails' 13 orientation to the relatively straight orientation required to significantly move the ureteral stent 1 through the bodily channel or lumen. Specifically, to remove the pigtails 13 from the bodily structure (such as the kidney or bladder), a threshold longitudinal pulling force may be required to urge the pigtails 13 into a relatively linear orientation required to extend through the tubular lumen. While the pigtail 13 is depicted, one of ordinary skill in the art will understand that the ends of the ureteral stent 1 may define other shapes, such as a J, a curve, or an angle, that may be used to retain the ends of the ureteral stent 1 in the kidney and bladder, respectively.

An outer surface of the first coiled wire 15 may be coated, such as with a fluoropolymer or other protective, lubricious coating, to produce a polymer coating 19. The polymer coating 19 may preferably be made of polyurethane, silicone, Teflon, or Pebax. In addition, the polymer coating 19 may be infused with preventive or other pharmacological compounds, bioactives, or biologics, such as heparin or other drugs. U.S. Publication Number 2013/0060238 entitled "Electrically Charged Medical Device" discloses a medical device with an outer surface prepared to reduce crystalline growth or surface fowling thereon. These features and other features disclosed in U.S. Publication Number 2013/0060238 could be used with the present invention. In other embodiments, Heparin may be coated onto the first (or second or both) coiled wire due to the heparin's ability to resist encrustation with long-term implantation of urinary tract medical devices. Fluoropolymers such as PTFE may help to enable the bonding of certain drugs, such as heparin, to the surface of the coils and therefore may be desirable in stents intended for long-term implantation. Other drugs useful for discouraging encrustation may include heparin, covalent heparin, dexamethazone, dexamethasone sodium phosphate, dexamethasone acetate and other dexamethasone derivatives, triclosan, silver nitrate, ofloxacin, ciproflaxin, phosphorylcholine, and triemethoprim. In other embodiments, the polymer coating 19 may be charged to establish a net negative charge on the outer surface of the ureteral stent 1 which is known to minimize encrustation.

In addition, one or more additional medications or drugs may be infused into the polymer coating 19 in order to assist in patient care and comfort. For instance, an antimicrobial drug, such as a combination of rifampin and minocycline, may help to reduce inflammation and microbial activity in the vicinity of the ureteral stent 1. Antimicrobial drugs infused into the polymer coating 19 may include the following drugs, or their salts or derivatives: rifampin, minocycline, a mixture of rifampin and minocycline, a non-steroidal anti-inflammatory agent, a penicillin, a cephalosporin, a carbepenem, a beta-lactam, an antibiotic, an aminoglycoside, a macrolide, a lincosamide, a glycopeptide, a tetracyline, a chloramphenicol, a quinolone, a fucidin, a sulfonamide, a trimethoprim, a rifamycin, an oxaline, a streptogramin, a lipopeptide, a ketolide, a polyene, an azole, an echinocandin, alpha-terpineol, methylisothiazolone, cetylpyridinium chloride, chloroxyleneol, hexachlorophene, chlorhexidine and other cationic biguanides, methylene chloride, iodine and iodophores, triclosan, taurinamides, nitrofurantoin, methenamine, aldehydes, azylic acid, rifampycin, silver, benzyl peroxide, alcohols, carboxylic acids and salts, and silver sulfadiazine. Also useful as antimicrobials are anthracyclines, such as doxorubicin or mitoxantrone, fluoropyrimidines such as 5-fluoroacil, and also podophylotoxins, such as etoposide. The salts and the derivatives of all of these are meant to be included as examples of antimicrobial drugs.

Analgesics, such as aspirin or other non-steroidal anti-inflammatory drugs, may also be infused into the polymer coating 19 to reduce pain and swelling upon implantation of the ureteral stent 1. These drugs or their salts or derivatives may include aspirin and non-steroidal anti-inflammatory drugs, including naproxen, choline, diflunisal, salsalate, fenoprofen, flurbiprofen, ketoprofen, ibuprofen, oxaprozin, diclofenac, indomethacin, sulindac, acetoaminophen, tolmetin, meloxicam, piroxicam, meclofenamate, mefanimic acid, nabumetone, etodelac, keterolac, celecoxib, valdecoxib, rofecoxib, mixtures thereof, and derivatives thereof.

Other analgesics or anesthetics that may be infused into the polymer coating 19 include opioids, synthetic drugs with narcotic properties, and local anesthetics to include at least paracetamol, bupivacaine, ropivacaine, lidocaine, novacaine, alfentanil, buprenorphine, carfentanil, codeine, codeinone, dextropropoxyphene, dihydrocodeine, endorphin, fentanyl, hydrocodone, hydromorphone, methadone, morphine, morphinone, oxycodone, oxymorphone, pethidine, remifantanil, sulfentanil, thebaine, tramadol, mixtures thereof, and derivatives thereof.

Any of these drugs and coatings may preferably be applied in a time-release manner so that the beneficial effect of the drug may be sustained over a period of at least several weeks or months. This may be especially helpful in the case where the ureteral stent 1 will remain in place for a considerable length of time.

The plurality of apertures 9 are located within the distal end portion 3, the proximal end portion 5, and the central portion 7 of the ureteral stent 1. Each of the plurality of apertures 9 pass through the polymer coating 19, the first coiled wire 15, the second coiled wire 17, and into the second lumen 11. Fluid, such as urine or bile, may flow through the plurality of apertures 9 and into the second lumen 11. The plurality of apertures 9 may be produced by means of a laser burn through the polymer coating 19, the first coiled wire 15, and the second coiled wire 17. The plurality of apertures 9 in each layer (the polymer coating 19, the first coiled wire 15, the second coiled wire 17) may be burnt at the same time or at separate times. Other methods known to produce the plurality of apertures 9, such as drilling, may be used.

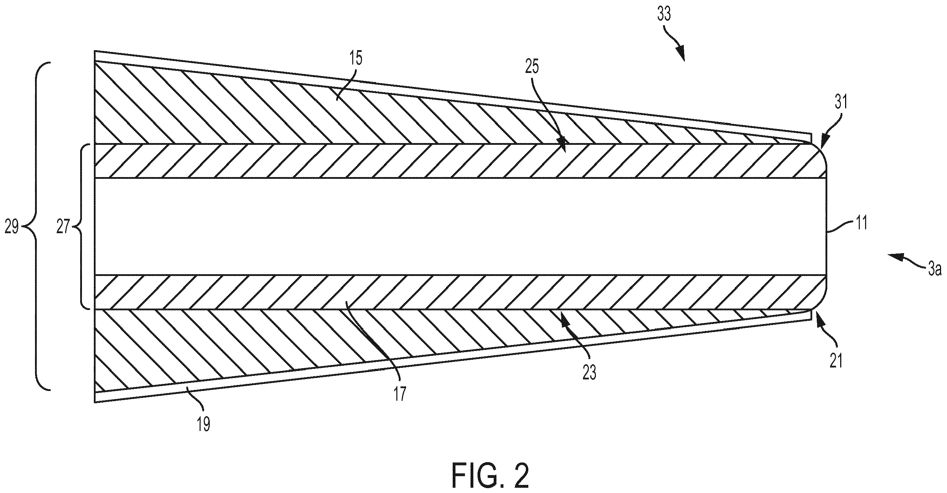

In FIG. 2, a tapered tip 33 of the distal end portion 3 is shown. A tapered tip 35 of the proximal end portion 5 when provided may be similar. The tapered tip 33 of the distal end portion 3, the tapered tip 35 of the proximal end portion 5, or both tapered tips 33, 35 may be provided. The first coiled wire 15 may have an inner diameter 27 and an outer diameter 29, where the outer diameter 29 is larger than the inner diameter 27. The outer diameter 29 may preferably be about 1.67 mm (5 Fr) to about 2.67 mm (8 Fr). Other diameters may be used. To form the tapered tip 33 of the distal end portion 3, the outer diameter 29 may taper to reduce the outer diameter 29 toward a distal end tip 3a of the distal end portion 3 while the inner diameter 27 may remain constant. To form the tapered tip 35 of the proximal end portion 5, the outer diameter 29 may taper to reduce the outer diameter 29 toward a proximal end tip 5a of the proximal end portion 5 while the inner diameter 27 may remain constant. In some embodiments, the outer diameter 29 at the end tips 3a, 5a may preferably be about 0.67 mm (2 Fr) to about 1.67 mm (5 Fr), or may preferably be about 1 mm (3 Fr) less than the nominal outer diameter 29 of the remainder of the ureteral stent 1. The taper of the outer diameter 29 may be formed by means of outer diameter step grinding or straight cutting. Other methods of tapering the outer diameter 29 may be used. Additionally, the end of the tapered tip 33 of the distal end portion 3 may have a laser welded transition 31 between the first coiled wire 15 and the second coiled wire 17 to form a smooth transition. The laser welded transition 31 may provide for relatively atraumatic distal and proximal end portions 3, 5 to allow for gradually dilating the neighboring tissue when the ureteral stent 1 is inserted into the proper position.

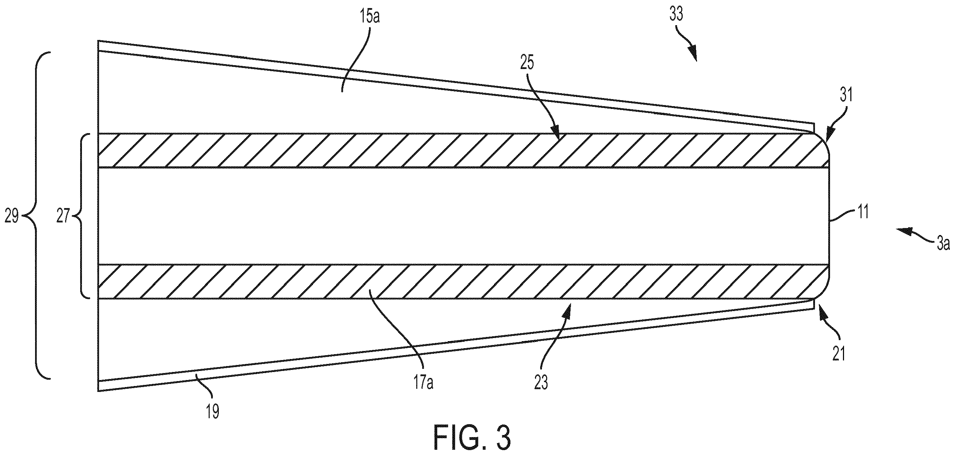

In FIG. 3, an ureteral stent not forming part of the invention is shown. In this example, the ureteral stent 1 may be made from an outer sleeve 15a and an inner coiled wire 17a disposed within a first lumen 21 defined by the outer sleeve 15a. The outer sleeve 15a may define a distal end portion 3, a proximal end portion 5, and a central portion 7 between the two end portions 3, 5. The outer sleeve 15a and inner coiled wire 17a may be manufactured from any material, and bio-compatible materials may be preferred. The outer sleeve 15a and inner coiled wire 17a may each be manufactured from the same material or different materials. The following materials may be preferable: 302 stainless steel, MP35N, 35N LT, CP Titanium, platinum alloys, 304V stainless steel, DFT, Ti6Al-4V ELI titanium alloy, 316L stainless steel, L-605, and nitinol.

The inner coiled wire 17a may define a distal end portion 3, a proximal end portion 5, and a central portion 7 between the two end portions 3, 5, which may be aligned with the respective distal and proximal end portions 3, 5 and central portion 7 of the outer sleeve 15a. The inner coiled wire 17a may define a second lumen 11 therewithin.

The outer sleeve 15a and inner coiled wire 17a may be secured together such that substantially all of an outer surface 25 of the inner coiled wire 17a may contact substantially all of an inner surface 23 of the outer sleeve 15a so that the inner coiled wire 17a may be fixedly retained within first lumen 21. In the context of the outer surface 25 of the inner coiled wire 17a contacting the inner surface 23 of the outer sleeve 15a, "substantially" is defined as at least 75% of the surface area of the outer surface 25 of the inner coiled wire 17a contacting the inner surface 23 of the outer sleeve 15a. The outer sleeve 15a and inner coiled wire 17a may be longitudinally fixed together. The outer sleeve 15a and inner coiled wire 17a may be fixed together with one or more weld beads, mechanical fasteners, adhesives, press fit, or by other methods known to fix similar materials together. In some embodiments, the inner coiled wire 17a may be a flat wire or a round wire. Furthermore, the inner coiled wire 17a may be hollow.

An outer surface of the outer sleeve 15a may be coated, such as with a fluoropolymer or other protective, lubricious coating, to produce a polymer coating 19. The polymer coating 19 may preferably be made of polyurethane, silicone, Teflon, or Pebax. In addition, the polymer coating 19 may be infused with preventive or other pharmacological compounds, bioactives, or biologics, as described above.

The plurality of apertures 9 may be located within one of more of the distal end portion 3, the proximal end portion 5, and the central portion 7 of the ureteral stent 1. Each of the plurality of apertures 9 may pass through the polymer coating 19, the outer sleeve 15a, the inner coiled wire 17a, and into the second lumen 11. Fluid, such as urine or bile, may flow through the plurality of apertures 9 and into the second lumen 11. The plurality of apertures 9 may be produced by means of a laser burn through the polymer coating 19, the outer sleeve 15a, and the inner coiled wire 17a. The plurality of apertures 9 in each layer (the polymer coating 19, the outer sleeve 15a, the inner coiled wire 17a) may be burnt at the same time or at separate times. Other methods known to produce the plurality of apertures 9, such as drilling, may be used.

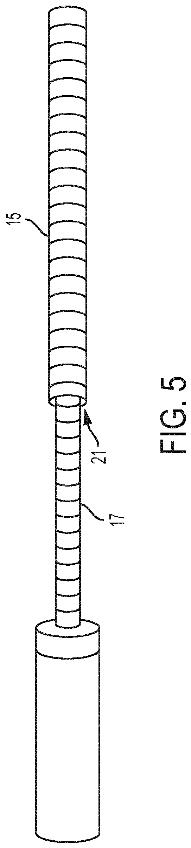

In FIG 5, the first and second coiled wires 15, 17 of an ureteral stent are shown. The second coiled wire 17 is disposed within a first lumen 21 defined by the first coiled wire 15. The first coiled wire 15 may be wound in a first helical direction along its length, and the second coiled wire 17 may be wound in a second helical direction along its length. The first and second helical directions may be the same or opposite to each other.

While the present stent may be highly useful for drainage of the kidneys, similar stents may be used in other hollow parts of the body. These may include biliary or gall bladder stents, stents for use in percutaneous nephrostomy procedures, hepatic drainage, gastrointestinal drainage, and so on, for drainage of other body cavities. It is intended that the foregoing detailed description be regarded as illustrative rather than limiting, and that it be understood that it is the following claims, including all equivalents, that are intended to define the scope of this invention.

## Claims

1. A stent comprising:
a first coiled wire (15) defining a first lumen (21) defined by an inner surface collectively of each coil formed from the first coiled wire, and the first coiled wire comprising a distal end portion, a proximal end portion, and a central portion therebetween;
a second coiled wire (17) defining an outer surface collectively of each coil formed by the second coiled wire, the second coiled wire defining a second lumen (11), the second coiled wire disposed within the first lumen of the first coiled wire and secured to the first coiled wire such that substantially the entire outer surface of the second coiled wire contacts substantially the entire inner surface of the first coiled wire, the second coiled wire comprising a distal end portion, a proximal end portion, and a central portion therebetween, the first and second coiled wires aligned with respect to each other so that the respective distal end portions, proximal end portions, and central portions of the first and second coiled wires are disposed proximate to each other; and
a plurality of apertures (9) passing through each of the first and second coiled wires and into the second lumen, the plurality of apertures located within the distal end, proximal end, and central portions of the first and second coiled wires.

2. The stent of claim 1 wherein the distal and proximal end portions of the first and second coiled wires are open.

3. The stent of any preceding claim, wherein the first and second coiled wires are flat wires.

4. The stent of claims 1 or 2, wherein the first and second coiled wires are round wires.

5. The stent of either of claims 1 or 2, wherein the first coiled wire is one of a flat or round wire and the second coiled wire is the other of the flat or the round wire.

6. The stent of any preceding claim, further comprising at least one pigtail defined by the distal end portion, the proximal end portion, or both end portions of the first and second coiled wires.

7. The stent of any one of claims 1, 2, 3, 4, or 5, further comprising at least one end defined by the distal end portion, the proximal end portion, or both end portions of the first and second coiled wires, wherein the at least one end has a shape of one of a J, a curve, or an angle.

8. The stent of any preceding claim, further comprising a polymer coating around an outer surface of the first coiled wire, wherein the plurality of apertures further passes through the polymer coating.

9. The stent of claim 8 wherein the polymer coating is infused with one of a pharmacological compound, a bioactive compound, or a biologic compound.

10. The stent of any preceding claim wherein the first coiled wire comprises an inner diameter and an outer diameter, the outer diameter being tapered at the distal end portion of the first coiled wire toward a distal end tip upon the distal end portion of the first coiled wire, and the inner diameter being constant at the distal end portion of the first coiled wire.

11. The stent of claim 10, wherein the outer diameter of the first coiled wire is tapered at the proximal end portion of the first coiled wire toward a proximal end tip upon the proximal end portion of the first coiled wire, and the inner diameter of the first coiled wire is constant at the proximal end portion of the first coiled wire.

12. The stent of any preceding claim, further comprising a laser welded transition between the first coiled wire and the second coiled wire.

13. The stent of any preceding claim, wherein the first coiled wire is wound in a first helical direction along its length, and the second coiled wire is wound in a second helical direction along its length, wherein the first and second helical directions are opposite to each other.

14. The stent of any preceding claim, wherein the first and second coiled wires are longitudinally fixed with respect to each other.

## Patentansprüche

1. Stent, umfassend:
einen ersten gewickelten Draht (15), der ein erstes Lumen (21) definiert, das von einer Innenfläche gemeinsam von jeder aus dem ersten gewickelten Draht ausgebildeten Spirale definiert wird, und wobei der erste gewickelte Draht einen distalen Endabschnitt, einen proximalen Endabschnitt und einen mittleren Abschnitt dazwischen umfasst;
einen zweiten gewickelten Draht (17), der eine Außenfläche gemeinsam von jeder aus dem zweiten gewickelten Draht ausgebildeten Spirale definiert, wobei der zweite gewickelte Draht ein zweites Lumen (11) definiert, der zweite gewickelte Draht im ersten Lumen des ersten gewickelten Drahts angeordnet ist und derart am ersten gewickelten Draht befestigt ist, dass im Wesentlichen die gesamte Außenfläche des zweiten gewickelten Drahts im Wesentlichen die gesamte Innenfläche des ersten gewickelten Drahts berührt, der zweite gewickelte Draht einen distalen Endabschnitt, einen proximalen Endabschnitt und einen mittleren Abschnitt dazwischen umfasst, der erste und der zweite gewickelte Draht zueinander ausgerichtet sind, so dass die jeweiligen distalen Endabschnitte, proximalen Endabschnitte und mittleren Abschnitte des ersten und des zweiten gewickelten Drahts nebeneinander angeordnet sind; und
eine Vielzahl von Öffnungen (9), die jeweils durch den ersten und den zweiten gewickelten Draht und in das zweite Lumen verlaufen, wobei die Vielzahl von Öffnungen in den distalen Endabschnitten, proximalen Endabschnitten und mittleren Abschnitten des ersten und des zweiten gewickelten Drahts angeordnet sind.

2. Stent nach Anspruch 1, wobei die distalen und proximalen Endabschnitte des ersten und des zweiten gewickelten Drahts offen sind.

3. Stent nach einem der vorhergehenden Ansprüche, wobei der erste und der zweite gewickelte Draht ein flacher Draht ist.

4. Stent nach Anspruch 1 oder 2, wobei der erste und der zweite gewickelte Draht ein runder Draht ist.

5. Stent nach Anspruch 1 oder 2, wobei der erste gewickelte Draht einer von einem flachen oder runden Draht ist und der zweite gewickelte Draht der jeweils andere des flachen oder runden Drahts ist.

6. Stent nach einem der vorhergehenden Ansprüche, ferner umfassend mindestens einen Pigtail, der vom distalen Endabschnitt, vom proximalen Endabschnitt oder von beiden Endabschnitten des ersten und des zweiten gewickelten Drahts definiert wird.

7. Stent nach einem der Ansprüche 1, 2, 3, 4 oder 5, ferner umfassend mindestens ein Ende, das vom distalen Endabschnitt, vom proximalen Endabschnitt oder von beiden Endabschnitten des ersten und zweiten gewickelten Drahts definiert wird, wobei das mindestens eine Ende eine Form eines J, einer Kurve oder eines Winkels aufweist.

8. Stent nach einem der vorhergehenden Ansprüche, ferner umfassend eine Polymerbeschichtung um eine Außenfläche des ersten gewickelten Drahts, wobei die Vielzahl von Öffnungen ferner durch die Polymerbeschichtung verläuft.

9. Stent nach Anspruch 8, wobei die Polymerbeschichtung mit einer von einer pharmakologischen Verbindung, einer bioaktiven Verbindung oder einer biologischen Verbindung durchsetzt ist.

10. Stent nach einem der vorhergehenden Ansprüche, wobei der erste gewickelte Draht einen Innendurchmesser und einen Außendurchmesser umfasst, wobei der Außendurchmesser am distalen Endabschnitt des ersten gewickelten Drahts zu einer distalen Endspitze auf dem distalen Endabschnitt des ersten gewickelten Drahts verjüngt ist und der Innendurchmesser am distalen Endabschnitt des ersten gewickelten Drahts konstant ist.

11. Stent nach Anspruch 10, wobei der Außendurchmesser des ersten gewickelten Drahts am proximalen Endabschnitt des ersten gewickelten Drahts zu einer proximalen Endspitze auf dem proximalen Endabschnitt des ersten gewickelten Drahts verjüngt ist und der Innendurchmesser des ersten gewickelten Drahts am proximalen Endabschnitt des ersten gewickelten Drahts konstant ist.

12. Stent nach einem der vorhergehenden Ansprüche, ferner umfassend einen lasergeschweißten Übergang zwischen dem ersten gewickelten Draht und dem zweiten gewickelten Draht.

13. Stent nach einem der vorhergehenden Ansprüche, wobei der erste gewickelte Draht in einer ersten spiralförmigen Richtung entlang seiner Länge gewickelt ist und der zweite gewickelte Draht in einer zweiten spiralförmigen Richtung entlang seiner Länge gewickelt ist, wobei die erste und die zweite spiralförmige Richtung einander gegenüberliegen.

14. Stent nach einem der vorhergehenden Ansprüche, wobei der erste und der zweite gewickelte Draht längs bezüglich einander fixiert sind.

## Revendications

1. Stent, comprenant:
un premier fil spiralé (15) qui définit une première lumière (21) définie par une surface intérieure collectivement de chaque bobine formée à partir du premier fil spiralé, et le premier fil spiralé comprenant une partie d'extrémité distale, une partie d'extrémité proximale et une partie centrale entre celles-ci;
un second fil spiralé (17) qui définit une surface extérieure collectivement de chaque bobine formée par le second fil spiralé, le second fil spiralé définissant une seconde lumière (11), le second fil spiralé étant disposé à l'intérieur de la première lumière du premier fil spiralé et étant fixé au premier fil spiralé de telle sorte que sensiblement la totalité de la surface extérieure du second fil spiralé entre en contact avec sensiblement la totalité de la surface intérieure du premier fil spiralé, le second fil spiralé comprenant une partie d'extrémité distale, une partie d'extrémité proximale et une partie centrale entre celles-ci, les premier et second fils spiralés étant alignés l'un par rapport à l'autre de telle sorte que les parties d'extrémité distales, les parties d'extrémité proximales et les parties centrales respectives des premier et second fils spiralés soient disposées à proximités les unes des autres; et
une pluralité d'ouvertures (9) qui passent à travers chacun des premier et second fils spiralés et dans la seconde lumière, la pluralité d'ouvertures étant situées à l'intérieur de l'extrémité distale, de l'extrémité proximale et des partie centrales des premier et second fils spiralés.

2. Stent selon la revendication 1, dans lequel les parties d'extrémité distale et proximale des premier et second fils spiralés sont ouvertes.

3. Stent selon l'une quelconque des revendications précédentes, dans lequel les premier et second fils spiralés sont des fils plats.

4. Stent selon la revendication 1 ou 2, dans lequel les premier et second fils spiralés sont des fils ronds.

5. Stent selon l'une ou l'autre des revendications 1 ou 2, dans lequel le premier fil spiralé est un parmi un fil plat et un fil rond, et le second fil spiralé est l'autre parmi le fil plat et le fil rond.

6. Stent selon l'une quelconque des revendications précédentes, comprenant en outre au moins un élément en tire-bouchon défini par la partie d'extrémité distale, la partie d'extrémité proximale ou les deux parties d'extrémité des premier et second fils spiralés.

7. Stent selon l'une quelconque des revendications 1, 2, 3, 4 ou 5, comprenant en outre au moins une extrémité définie par la partie d'extrémité distale, la partie d'extrémité proximale ou les deux parties d'extrémité des premier et second fils spiralés, dans lequel ladite au moins une extrémité a la forme d'un J, d'une courbe ou d'un angle.

8. Stent selon l'une quelconque des revendications précédentes, comprenant en outre un revêtement polymère autour d'une surface extérieure du premier fil spiralé, dans lequel la pluralité d'ouvertures passent en outre à travers le revêtement polymère.

9. Stent selon la revendication 8, dans lequel le revêtement polymère est infusé avec un parmi un composé pharmacologique, un composé bioactif ou un composé biologique.

10. Stent selon l'une quelconque des revendications précédentes, dans lequel le premier fil spiralé présente un diamètre intérieur et un diamètre extérieur, le diamètre extérieur étant conique à la partie d'extrémité distale du premier fil spiralé en direction d'une pointe d'extrémité distale sur la partie d'extrémité distale du premier fil spiralé, et le diamètre intérieur étant constant à la partie d'extrémité distale du premier fil spiralé.

11. Stent selon la revendication 10, dans lequel le diamètre extérieur du premier fil spiralé est conique à la partie d'extrémité proximale du premier fil spiralé en direction d'une pointe d'extrémité proximale sur la partie d'extrémité proximale du premier fil spiralé, et le diamètre intérieur du premier fil spiralé est constant à la partie d'extrémité proximale du premier fil spiralé.

12. Stent selon l'une quelconque des revendications précédentes, comprenant en outre une transition soudée au laser entre le premier fil spiralé et le second fil spiralé.

13. Stent selon l'une quelconque des revendications précédentes, dans lequel le premier fil spiralé est enroulé dans une première direction hélicoïdale le long de sa longueur, et le second fil spiralé est enroulé dans une seconde direction hélicoïdale le long de sa longueur, dans lequel les première et seconde directions hélicoïdales sont opposées l'une à l'autre.

14. Stent selon l'une quelconque des revendications précédentes, dans lequel les premier et second fils spiralés sont fixés de façon longitudinale l'un par rapport à l'autre.
